# EUROPÄISCHE PATENTSCHRIFT

(11) **EP 2 484 350 B1**
(45) Veröffentlichungstag und Bekanntmachung des Hinweises auf die Patenterteilung: **20.04.2016**
(21) Anmeldenummer: 11153452.5
(22) Anmeldetag: 04.02.2011
(51) Int. Cl.: A61K 9/19, A61K 47/48, A61K 31/05

(54) **Pharmazeutische Zubereitung, enthaltend einen Komplex eines Propofolsalzes mit einem Cyclodextrin**
Pharmaceutical preparation containing a complex of a salt of propofol and a cyclodextrin
Préparation pharmaceutique comprenant un complexe d'un sel de propofol et d'une cyclodextrine

(43) Veröffentlichungstag der Anmeldung: 08.08.2012
(73) Patentinhaber: Roewer, Norbert, Univ.-Prof. Dr. med., 97082 Würzburg (DE); Broscheit, Jens, Dr. med., 97209 Würzburg (DE)
(72) Erfinder: Roewer, Norbert, Univ.-Prof. Dr. med., 97082 Würzburg (DE); Broscheit, Jens, Dr. med., 97209 Würzburg (DE)
(74) Vertreter: Glawe, Delfs, Moll

(56) Entgegenhaltungen:
- EP-A1- 2 106 786
- WO-A1-96/32135
- WO-A1-02/074200
- WO-A1-03/080079
- JARA F ET AL: "The interaction of solvatochromic pyridiniophenolates with cyclodextrins", TETRAHEDRON, ELSEVIER SCIENCE PUBLISHERS, AMSTERDAM, NL, Bd. 62, Nr. 33, 14. August 2006 (2006-08-14), Seiten 7817-7823, XP025002500, ISSN: 0040-4020, DOI: DOI:10.1016/J.TET.2006.05.053 [gefunden am 2006-08-14]
- EFTINK M R ET AL: "Calorimetric studies of p-nitrophenol binding to alpha- and beta-cyclodextrin", BIOORGANIC CHEMISTRY, ACADEMIC PRESS INC., NEW YORK, NY, US, Bd. 10, Nr. 4, 1. Dezember 1981 (1981-12-01), Seiten 388-398, XP024022681, ISSN: 0045-2068, DOI: DOI:10.1016/0045-2068(81)90051-1 [gefunden am 1981-12-01]

## Beschreibung

Die Erfindung betrifft eine pharmazeutische Formulierung, die einen Komplex eines Propofolsalzes mit 2-Hydroxypropyl-β-Cyclodextrin enthält.

In der Pharmazie ist es ein häufiges Problem, einen pharmazeutischen Wirkstoff so zu formulieren, dass er mittels einer bestimmten Applikationsart in der gewünschten Konzentration und möglichst effizient an den vorgesehenen Wirkort gebracht wird. So muss ein für intravenöse Applikation vorgesehener Wirkstoff in gewissem Umfang wasserlöslich sein, damit überhaupt eine systemische Konzentration im Blut erreicht werden kann. Andererseits muss er in der Regel eine gewisse Lipophilie aufweisen, um am vorgesehenen Wirkort ggf. Zellmembranen durchdringen zu können. Ein lediglich gut wasserlöslicher Wirkstoff kann beispielsweise nach intravenöser Applikation eine hohe systemische Konzentration im Blut aufbauen, er wird aber beispielsweise bei zu geringer Lipophilie eine geringe Bioverfügbarkeit aufweisen, da am vorgesehenen Wirkort möglicherweise ein unzureichender Durchtritt durch die Zellmembran erfolgt.

Propofol ist ein erstmals 1977 in der klinischen Praxis getestetes intravenöses Anästhetikum. Propofol ist ein kaum wasserlöslicher anästhetischer Wirkstoff, der einer intravenösen Administration zugänglich gemacht werden muss.

Die Lösung des Anästhetikums in einer Fettemulsion (Handelsname Diprivan®) konnte den häufig beobachteten Venenschmerz bei der i.v. Applikation vermindern, so dass Propofol 1989 in die klinische Praxis eingeführt wurde.

Diese Propofol-Lipidemulsion enthält Sojaöl, Glycerol und Eiphosphatide. Venenschmerz bei der Injektion ist ein nach wie vor häufig auftretendes Problem. Zudem kann es zu schwerwiegenden allergischen Reaktionen kommen. Da die Formulierung als Fettemulsion mikrobielles Wachstum begünstigt, kann es bei einer Kontamination der Emulsion bereits nach kurzen Lagerungszeiten zu einer Sepsis nach Applikation kommen.

WO 96/32135 beschreibt eine Formulierung enthaltend Propofol komplexiert mit 2-Hydroxypropyl-β-cyclodextrin in eine wässrigen Lösung.

Der Erfindung liegt die Aufgabe zugrunde, eine pharmazeutische Formulierung der eingangs genannten Art zu schaffen, die eine problemlose intravenöse Administration erlaubt und die genannten Nachteile nicht oder im geringeren Maße aufweist.

Gelöst wird diese Aufgabe dadurch, dass Propofol als Salz mit 2-Hydroxypropyl-β-Cyclodextrin komplexiert wird. Propofol kann in alkalischem Milieu in ein Salz umgesetzt werden. Das Propofol-Anion kann von dem Cyclodextrin komplexiert werden. Der Begriff Propofolsalz bezeichnet im Rahmen der Erfindung das Anion des Propofols (Phenolat), das von dem Cyclodextrin komplexiert werden kann. In dem erfindungsgemäßen Komplex liegt Propofol somit als Anion komplexiert vor.

Überraschenderweise hat sich gezeigt, dass sich auf diese Weise eine stabile, injizierbare, pharmazeutische Formulierung mit einer hohen Propofolkonzentration herstellen lässt. Die erfindungsgemäße pharmazeutische Formulierung kann als wässrige, zur Injektion geeignete Lösung zur Verfügung gestellt werden. Alternativ ist es möglich, nach der Herstellung des Komplexes das Lösungsmittel abzuziehen und den Komplex als lagerfähigen Feststoff zur Verfügung zu stellen. Vor einer Administration wird dieser Feststoff wieder in eine wässrige Lösung überführt.

Der pH-Wert einer wässrigen Lösung des Komplexes liegt vorzugsweise über 7, weiter vorzugsweise über 8, weiter vorzugsweise über 9. Bevorzugte Obergrenzen für den pH-Wert sind 11 oder 10. Besonders bevorzugt ist ein Bereich des pH-Wertes von 8-11, weiter vorzugsweise 9-11, weiter vorzugsweise 9-10. Sofern der Komplex als lagerfähiger Feststoff zur Verfügung gestellt wird, stellt sich nach dem Resolubilisieren in Wasser bevorzugt ein pH-Wert aus den genannten Bereichen ein.

Als Cyclodextrin zur Komplexierung des Propofols wird erfindungsgemäß 2-Hydroxypropyl-Beta-Cyclodextrin (HPBCD) verwendet. Als Propofolsalz wird bevorzugt ein Alkalimetallsalz, besonders bevorzugt eine Natriumsalz verwendet.

Das Molverhältnis von Propofolsalz und Cyclodextrin zueinander beträgt vorzugsweise 1:2 bis 1:6, weiter vorzugsweise 1:2 bis 1:4, weiter vorzugsweise etwa 1:2. Der Propofolsalzgehalt des Komplexes beträgt vorzugsweise etwa 4 bis 9 Gew.-%.

Gegenstand der Erfindung ist ferner ein Verfahren zur Herstellung einer erfindungsgemäßen pharmazeutischen Formulierung. Erfindungsgemäß wird zunächst eine alkalische wässrige Lösung des Cyclodextrin hergestellt. In diese alkalische Lösung wird Propofol gegeben und vorzugweise unter Rühren bis zur Lösung und Komplexbildung vermischt. Dies geschieht bevorzugt unter Inertgasatmosphäre. Es ist erfindungsgemäß gleichfalls möglich, erst nach Zugabe des Propofols zu dem Cyclodextrin alkalische Bedingungen einzustellen.

Das Lösen und Komplexieren des Propofols geschieht vorzugsweise über einen Zeitraum von 2 bis 10 h, weiter vorzugsweise 3 bis 5 h, weiter vorzugsweise etwa 4 h. Erfindungsgemäß erfolgt der Löse- und Komplexbildungsvorgang wesentlich schneller als bei der Komplexierung der phenolischen Form von Propofol. Auch lässt sich eine deutlich höhere Konzentration vom Propofol im Komplex einstellen.

Die erfindungsgemäß erhaltene Lösung kann in einem weiteren Schritt von verbleibenden Feststoffanteilen befreit werden, beispielsweise filtriert werden. Geeignet ist z. B. eine Filtration durch einen Porenfilter mit der Porengröße 0,45 µm.

Aus der erfindungsgemäß hergestellten Lösung kann das Lösungsmittel durch bekannte und geeignete Verfahren wie beispielsweise Gefriertrocknung abgezogen werden. Der Komplex wird auf diese Weise als lagerfähiger und resolubilisierbarer Feststoff zur Verfügung gestellt.

Ein Ausführungsbeispiel der Erfindung wird nachfolgend erläutert.

Die Spezifikationen aller verwendeten Materialien entsprechen dem European Pharmacopeia (Ph.Eur.). Folgende Einsatzstoffe wurden verwendet:
- Propofol
- NaOH
- HPBCD
- Wasser für Injektionszwecke

NaOH wurde mit 60 ml Wasser zu einer 0,01 N NaOH-Lösung angesetzt. 12 g HPBCD wurden hinzu gegeben und unter Rühren gelöst. Anschließend wurden 0,817 g Propofol hinzu gegeben und für 4 h bei 40-80 min⁻¹ weiter gerührt, bis das entstehende Propofolsalz gelöst und komplexiert war. Die entstandene Lösung hat einen pH-Wert von 9-10.

Die erhaltene Lösung wurde durch einen Porenfilter mit einer Porengröße von 0,45 µm filtriert. Nach der Filtration wurde die Lösung lyophilisiert. Man erhielt ein weißes Pulver. Der erhaltene Komplex kann wie folgt charakterisiert werden:

| | |
|---|---|
| Wassergehalt: | 1,4 Gew.-% |
| pH-Wert einer 1%igen wässrigen Lösung: | 9,5 |
| Propofolgehalt (berechnet als aktive Phenolform): | 6,5 Gew.-% |
| Löslichkeit des Komplexes in Wasser: | 41 Gew.-% |

Nach Resolubilisation des Komplexes in Wasser erhält man eine wässrige Lösung mit einem Gehalt von aktivem Propofol von 46 mg/ml.

## Patentansprüche

1. Pharmazeutische Formulierung, die einen Komplex eines Propofolsalzes mit 2-Hydroxypropyl-β-Cyclodextrin (HPBCD) enthält.

2. Formulierung nach Anspruch 1, **dadurch gekennzeichnet, dass** das Propofolsalz ein Alkalimetallsalz ist.

3. Formulierung nach Anspruch 2, **dadurch gekennzeichnet, dass** das Propofolsalz ein Natriumsalz ist.

4. Formulierung nach einem der Ansprüche 1 bis 3, **dadurch gekennzeichnet, dass** das Molverhältnis Propofolsalz:Cyclodextrin 1:2 bis 1:6, vorzugsweise 1:2 bis 1:4, weiter vorzugsweise etwa 1:2 beträgt.

5. Formulierung nach einem der Ansprüche 1 bis 3, **dadurch gekennzeichnet, dass** der Propofolsalzgehalt des Komplexes 4 bis 9 Gew.-% beträgt.

6. Formulierung nach einem der Ansprüche 1 bis 5, **dadurch gekennzeichnet, dass** es eine zur Injektion geeignete wässrige Lösung ist.

7. Formulierung nach Anspruch 6, **dadurch gekennzeichnet, dass** der pH-Wert zwischen 7 und 11, vorzugsweise zwischen 8 und 11, weiter vorzugsweise zwischen 9 und 11, weiter vorzugsweise zwischen 9 und 10 liegt.

8. Formulierung nach einem der Ansprüche 1 bis 5, **dadurch gekennzeichnet, dass** es ein wasserlöslicher Feststoff ist.

9. Formulierung nach einem der Ansprüche 1 bis 8 zur Verwendung als Anästhetikum.

10. Verfahren zur Herstellung einer Formulierung nach einem der Ansprüche 1 bis 7, **gekennzeichnet durch** die Schritte:
a) Herstellen einer alkalischen wässrigen Lösung des Cyclodextrins,
b) Lösen von Propofol in der alkalischen wässrigen Lösung.

11. Verfahren nach Anspruch 10, **dadurch gekennzeichnet, dass** das Lösen des Propofols unter Inertgasatmosphäre stattfindet.

12. Verfahren nach Anspruch 10 oder 11, **dadurch gekennzeichnet, dass** das Lösen des Propofols über einen Zeitraum von 2 bis 10 h, vorzugsweise 3 bis 5 h, weiter vorzugsweise etwa 4 h erfolgt.

13. Verfahren nach einem der Ansprüche 10 bis 12, **dadurch gekennzeichnet, dass** die in Schritt b) erhaltene Lösung filtriert wird.

14. Verfahren nach einem der Ansprüche 10 bis 13, **dadurch gekennzeichnet, dass** aus der in Schritt b) erhaltenen und ggf. filtrierten Lösung Wasser entfernt wird, vorzugsweise durch Gefriertrocknung entfernt wird.

## Claims

1. Pharmaceutical formulation containing a complex of a propofol salt comprising 2-hydroxypropyl-β-cyclodextrin (HPBCD).

2. Formulation according to claim 1, **characterised in that** the propofol salt is an alkali metal salt.

3. Formulation according to claim 2, **characterised in that** the propofol salt is a sodium salt.

4. Formulation according to any of claims 1 to 3, **characterised in that** the molar ratio of propofol salt to cyclodextrin is from 1:2 to 1:6, preferably from 1:2 to 1:4, more preferably approximately 1:2.

5. Formulation according to any of claims 1 to 3, **characterised in that** the content of propofol salt in the complex is from 4 to 9 wt.%.

6. Formulation according to any of claims 1 to 5, **characterised in that** said formulation is an aqueous solution suitable for injection.

7. Formulation according to claim 6, **characterised in that** the pH of said formulation is between 7 and 11, preferably between 8 and 11, more preferably between 9 and 11, and even more preferably between 9 and 10.

8. Formulation according to any of claims 1 to 5, **characterised in that** said formulation is a water-soluble solid.

9. Formulation according to any of claims 1 to 8 for use as an anaesthetic.

10. Method for producing a formulation according to any of claims 1 to 7, **characterised by** the following steps:
a) producing an alkaline aqueous cyclodextrin solution,
b) dissolving propofol in the alkaline aqueous solution.

11. Method according to claim 10, **characterised in that** the propofol is dissolved under an inert gas atmosphere.

12. Method according to either claim 10 or claim 11, **characterised in that** the propofol is dissolved over a period of from 2 to 10 hours, preferably from 3 to 5 hours, more preferably approximately 4 hours.

13. Method according to any of claims 10 to 12, **characterised in that** the solution obtained in step b) is filtered.

14. Method according to any of claims 10 to 13, **characterised in that** water is removed, preferably by means of freeze drying, from the solution which is obtained in step b) and optionally filtered.

## Revendications

1. Formulation pharmaceutique qui contient un complexe d'un sel de propofol avec de la 2-hydroxypropyl-β-cyclodextrine (HPBCD).

2. Formulation selon la revendication 1, **caractérisée en ce que** le sel de propofol est un sel d'un métal alcalin.

3. Formulation selon la revendication 2, **caractérisée en ce que** le sel de propofol est un sel de sodium.

4. Formulation selon l'une des revendications 1 à 3, **caractérisée en ce que** le rapport en moles sel de propofol:cyclodextrine est de 1:2 à 1:6, de préférence de 1:2 à 1:4, d'une manière plus préférée d'environ 1:2.

5. Formulation selon l'une des revendications 1 à 3, **caractérisée en ce que** la teneur en propofol du complexe est de 4 à 9 % en poids.

6. Formulation selon l'une des revendications 1 à 5, **caractérisée en ce qu'**il s'agit d'une solution aqueuse convenant à une injection.

7. Formulation selon la revendication 6, **caractérisée en ce que** le pH est compris entre 7 et 11, de préférence entre 8 et 11, d'une manière plus préférée entre 9 et 11, d'une manière plus préférée entre 9 et 10.

8. Formulation selon l'une des revendications 1 à 5, **caractérisée en ce qu'**il s'agit d'un solide soluble dans l'eau.

9. Formulation selon l'une des revendications 1 à 8 pour une utilisation en tant qu'anesthésiant.

10. Procédé de préparation d'une formulation selon l'une des revendications 1 à 7, **caractérisé par** les étapes :
a) préparation d'une solution aqueuse alcaline de cyclodextrine,
b) dissolution de propofol dans la solution aqueuse alcaline.

11. Procédé selon la revendication 10, **caractérisé en ce que** la dissolution du propofol a lieu sous une atmosphère d'un gaz inerte.

12. Procédé selon la revendication 10 ou 11, **caractérisé en ce que** la dissolution du propofol a lieu sur une durée de 2 à 10 h, de préférence de 3 à 5 h, d'une manière plus préférée d'environ 4 h.

13. Procédé selon l'une des revendications 10 à 12, **caractérisé en ce que** la solution obtenue dans l'étape b) est soumise à une filtration.

14. Procédé selon l'une des revendications 10 à 13, **caractérisé en ce que** l'eau est éliminée, de préférence est éliminée par lyophilisation, de la solution obtenue dans l'étape b) et éventuellement filtrée.
